# EUROPÄISCHE PATENTSCHRIFT

(11) **EP 1 186 653 B1**
(45) Veröffentlichungstag und Bekanntmachung des Hinweises auf die Patenterteilung: **05.09.2012**
(21) Anmeldenummer: 01810718.5
(22) Anmeldetag: 19.07.2001
(51) Int. Cl.: C12M 3/02, C12M 1/40, C12M 1/36

(54) **Bioreaktor zum schwebenden Halten eines Gutes**
Bioreaktor to keep an article in suspension
Bioréacteur pour maintenir un objet en suspension

(30) Priorität: 18.08.2000 EP 00810734
(43) Veröffentlichungstag der Anmeldung: 13.03.2002
(73) Patentinhaber: Levitronix Technologies, LLC, Waltham MA 02451 (US)
(72) Erfinder: Schöb, Reto, Dr., 8604 Volketswil (CH)
(74) Vertreter: Sulzer Management AG

(56) Entgegenhaltungen:
- EP-A- 0 472 223
- EP-A- 1 087 010
- WO-A-86/00636
- US-A- 4 978 616
- VER LEAH MAY B ET AL: "Design criteria of a fluidized bed oyster nursery." AQUACULTURAL ENGINEERING, Bd. 14, Nr. 3, 1995, Seiten 229-249, XP000982532 ISSN: 0144-8609
- MARUYAMA T ET AL: "LIQUID FLUIDIZATION IN CONICAL VESSELS" CHEMICAL ENGINEERING JOURNAL, Bd. 46, Nr. 1, 1991, Seiten 15-22, XP000982511 ISSN: 0923-0467

## Beschreibung

Die Erfindung betrifft ein Bioreaktor zum schwebenden Halten eines Gutes

Die erfindung ist nur durch die Ansprüche limitiert.

Das künstliche Herstellen von Gewebematerial, im Englischen als "Tissue Engineering" bezeichnet, gewinnt zunehmend an Bedeutung, um biologische Substitute für geschädigtes Gewebe oder geschädigte Organe herzustellen. Künstliches Gewebematerial ist herstellbar, indem Zellkulturen in vitro an oder in einem Gewebeträger, auch als Matrix bezeichnet, eingelagert werden. Der Gewebeträger besteht beispielsweise aus einem synthetischem Polymer oder aus einem biologischen Material wie Kollagen. Ein derartiger Gewebeträger wird auch als "Scaffold" bezeichnet. Die Zellen werden auf dem Gewebeträger ausgesät und beginnen sich, sofern die Umgebungsparameter physiologisch angepasst sind, zu vermehren. Der Gewebeträger kann derart ausgestaltet sein, dass dieser sich mit der Zeit auflöst, sodass nach einer bestimmten Zeit nur noch das aus dem Zellen gebildete Gewebeteil vorhanden ist. Der Gewebeträger und/oder das darauf gebildete Gewebeteil wird nachfolgend als "Gut" bezeichnet. Die für das Zellwachstum erforderlichen Bedingungen werden in einem Bioreaktor erzeugt, innerhalb welchem dem Gut der erforderliche Sauerstoff sowie ein Nährmedium zugeführt wird, und innerhalb welchem das Gut während mehreren Tagen bis Wochen verbleibt, bis die gewünscht Grösse erreicht ist. Die geometrische Form, welche das künstlich erzeugte Gewebematerial während dem Wachstum annimmt, wird wesentlich beeinflusst durch die Massnahmen, mit welchen das Gut im Bioreaktor gehalten wird.

Nachfolgend wird also unter dem Begriff "Gut" sowohl der Gewebeträger an sich, als auch der Gewebeträger mit angelagerten Zellen verstanden, oder falls der Gewebeträger abbaubar ausgestaltet ist, die künstlich erzeugte Zellkultur bzw. das künstlich erzeugte Gewebeteil.

Es ist Aufgabe ein Verfahren zum Halten eines Gutes in einem Bioreaktor vorzuschlagen, welches ein vorteilhaftes Wachstum ermöglicht. Es ist weiter Aufgabe einen Bioreaktor vorzuschlagen, welcher bezüglich dem Wachstum von Zellkulturen vorteilhafte Eigenschaften aufweist.

US 4978616 beschreibt einen bioreaktor mit einem Schwebenden gut, der als Fliessbettreaktor ausgebildet ist.

Die Aufgabe wird weiter gelöst mit einem Bioreaktor aufweisend die Merkmale von Ansprüchen 1,2 Die Unteransprüche 3 bis 20 betreffen weitere, vorteilhaft ausgestaltete Bioreaktoren.

Die Aufgabe wird insbesondere gelöst mit einem Verfahren zum schwebenden Halten eines Gutes in einem Bioreaktor, indem das Gut mit einem Fluid beaufschlag wird, und die Strömung des Fluides derart der Schwerkraft oder dem Auftrieb entgegengesetzt wirkt, dass das Gut in der Schwebe gehalten wird.

Das Verfahren weist den Vorteil auf, dass das Gut berührungslos im Bioreaktor gehalten wird, indem das Fluid, üblicherweise eine Flüssigkeit, eine derartig ausgebildete Strömung aufweist, dass das Gut von der entgegengesetzt der Schwerkraft wirkenden Strömung berührungslos gehalten wird. Dabei wird das Gut üblicherweise auch ständig in Bewegung gehalten, sodass sich dessen Lage ständig verändert. Das Verfahren weist den Vorteil auf, dass die Zellen gleichmässig am bzw. im Gut wachsen und das Wachstum des Guts begünstigt wird. Nachteilig an den bisher bekannten Verfahren zum künstlichen Herstellen von Gewebe ist die Tatsache, dass nur flache, im wesentlichen zweidimensionale Gebilde herstellbar waren.

In einem besonders vorteilhaft ausgestalteten Verfahren weist das Fluid in zur Schwerkraft entgegengesetzter Richtung eine zunehmend geringere Strömungsgeschwindigkeit auf. Dieses Strömungsverhalten wird beispielsweise dadurch erzeugt, dass das strömende Fluid von unten in einen sich gegen oben erweiternden, kegelstumpfförmigen Hohlkörper geleitet wird. Der sich gegen oben erweiternde Querschnitt des Hohlkörpers bewirkt, dass sich die Strömungsgeschwindigkeit im Hohlkörper mit zunehmender Höhe reduziert. Das Gut wird im Innenraum des Hohlkörpers ständig in der Schwebe gehalten, wobei die Seitenwände des Hohlkörpers eine seitliche Bewegung des Guts begrenzen, sodass sich das Gut ständig in der aufwärts strömenden Flüssigkeit befindet. Mit zunehmendem Zellwachstum erhöht sich das Gewicht des Guts, sodass das Gut im Innenraum des Hohlkörpers sich leicht nach unten bewegt und dort wieder eine neue Gleichgewichtslage finde. Das Gut sucht somit selbstständig die jeweilige Gleichgewichtslage. Es kann sich jedoch als vorteilhaft erweisen die Lage des Gutes mit einem Sensor zu überwachen und mit Hilfe des gemessenen Signals die Geschwindigkeit des aufströmenden Fluides zu beeinflussen. So kann die Geschwindigkeit des Fluides beispielsweise derart geregelt werden, dass das Gut ständig in einer vorbestimmten Lage in der Schwebe gehalten wird.

In einem vorteilhaften Verfahren wird nebst der Aufwärtsströmung innerhalb des Bioreaktors zudem eine Abwärtsströmung erzeugt, wobei dem abwärtsströmenden Fluid, üblicherweise eine Flüssigkeit, ein gasförmiges Fluid wie Luft oder Sauerstoff zugeleitet wird. Die Geschwindigkeit des abwärts strömenden Fluides wird vorteilhafterweise derart gewählt, dass das eingeleitete, gasförmige Fluid verlangsam oder gar nicht mehr aufsteigt, sodass das gasförmige Fluid relativ lange im strömenden Fluid verbleit und von diesem aufgenommen bzw. absorbiert werden kann.

Die Aufgabe wird weiter insbesondere gelöst mit einem Bioreaktor umfassend einen Behälter für ein mit einem Fluid zu beaufschlagendes Gut, wobei der Behälter einen ersten Strömungsraum umfasst, welchem ein strömendes Fluid zuführbar ist, und wobei der erste Strömungsraum derart ausgestaltet ist, dass das darin von unten nach oben strömende Fluid mit zunehmender Höhe eine kleinere Geschwindigkeit aufweist. In einer besonders vorteilhaften Ausgestaltung weist der Strömungsraum einen sich gegen oben erweiternden Querschnitt auf.

In einer weiteren vorteilhaften Ausgestaltung ist innerhalb des Bioreaktors ein Flussleitmittel angeordnet, welches einen sich von unten nach oben erweiternden Strömungsraum ausbildet. Vorzugsweise bildet dieses Flussleitmittel innerhalb des Bioreaktors zudem einen weiteren, sich von oben nach unten erweiternden zweiten Strömungsraum aus, in welchen ein gasförmiges Fluid einleitbar ist.

In einer weiteren, vorteilhaften Ausgestaltung ist innerhalb des Bioreaktors ein antreibbares Pumpenrad angeordnet, mit dessen Hilfe innerhalb des Bioreaktors die Strömung des Fluides erzeugbar ist. Vorteilhafterweise ist das Pumpenrad magnetisch an einen ausserhalb des Gehäuses des Bioreaktors angeordneten Antrieb gekoppelt. Das Bioreaktorgehäuse sowie das Pumpenrad sind vorteilhafterweise als Einweg- bzw. als Einmalprodukt konzipiert, sodass diese nach einer einmaligen Verwendung entsorgt werden. Diese Teile sind kostengünstig herstellbar. Beispielsweise umfass das Pumpenrad ein Flügelrad aus Kunststoff, in welchem ein Permanentmagnet eingegossen ist. Alle kostspieligen Komponenten wie die Antriebsvorrichtung sind ausserhalb des Bioreaktors angeordnet. Die Ausgestaltung des Bioreaktors als Einmalprodukt weist den Vorteil auf, dass kein aufwendiger Reinigungsprozess erforderlich ist, und dass eine Kontamination der künstlich hergestellten Gewebematerials weitgehend ausgeschlossen ist. Das Vermeiden einer Kontamination ist von entscheidender Bedeutung, da das Gut beispielsweise 4 bis 8 Wochen im Bioreaktor verbleibt, bis genügend künstliches Gewebematerial gebildet ist. Da der Bioreaktor über kein Immunsystem verfügt, können bereits kleinste Verunreinigungen wie Bakterien, Pilze oder Viren zur Folge haben, dass das hergestellte künstliche Gewebe abstirbt oder kontaminiert wird. Durch die Ausgestaltung des Bioreaktors als Einmalprodukt ist künstliches Gewebematerial kostengünstig und zuverlässig herstellbar.

Die Erfindung wird nachfolgend an Hand mehrerer Ausführungsbeispiele beschrieben. Es zeigen:
Fig. 1 einen Längsschnitt durch einen ersten Bioreaktor;
Fig. 1a eine perspektivische Detailansicht des Flussleitmittels;
Fig. 2a, 2b Längsschnitte durch weitere Ausführungsbeispiele von Bioreaktoren;
Fig. 3a -3d Längsschnitte durch weitere Ausführungsbeispiele von Bioreaktoren;
Fig. 4 einen Längsschnitt durch einen weiteren Bioreaktor mit einem magnetisch gekoppelten Flügelrad entlang der Linie B-B;
Fig. 5 einen Schnitt durch Figur 4 entlang der Linie A-A;
Fig. 6 einen Längsschnitt durch einen weiteren Bioreaktor mit einem an der verschliessbaren Öffnung angeordneten Flügelrad;
Fig. 7 einen Längsschnitt durch einen weiteren Bioreaktor mit einem magnetisch gelagerten Flügelrad entlang der Linie D-D;
Fig. 8 einen Schnitt durch Figur 7 entlang der Linie C-C;
Fig. 9 einen Längsschnitt durch einen weiteren Bioreaktor;
Fig. 10 einen Längsschnitt durch einen weiteren Bioreaktor;
Fig. 11 einen Längsschnitt durch einen weiteren Bioreaktor.

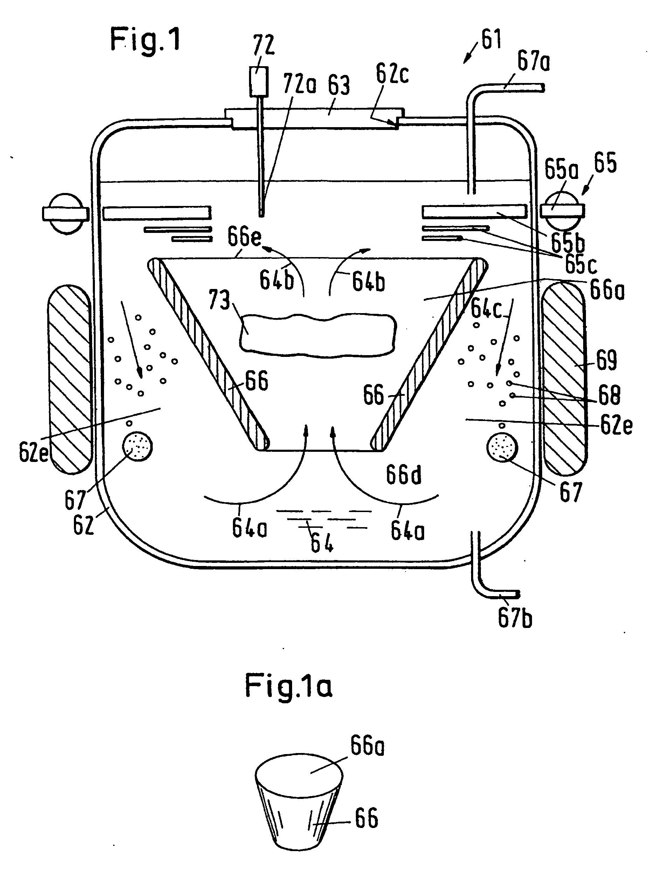

Der in Fig. 1 dargestellte Bioreaktor 61 umfasst einen Behälter 62, welcher oben eine mit einem Verschluss 63 verschliessbare Öffnung 62c aufweist. Im Innenraum des Behälter 62 ist ein kegelstumpfförmig, als Hohlkörper ausgebildetes Flussleitmittel 66 angeordnet, dessen Querschnittsfläche sich gegen oben vergrössert. Der Innenraum des Behälters 62 ist weitgehend mit einer Flüssigkeit 64 gefüllt, welche vom Flügelrad 65c des Motors 65 in eine Zirkulationsströmung versetzt wird, sodass die Flüssigkeit 64 die mit den Pfeilen 64a, 64b, 64c dargestellte Strömungsrichtung aufweist. Die in Richtung der Pfeile 64a strömende Flüssigkeit tritt von unten mit relativ hoher Flussgeschwindigkeit über die Eintrittsöffnung 66d in den Innenraum 66a des Flussleitmittels 66 ein, strömt im Innenraum 66a mit abnehmender Geschwindigkeit nach oben, und tritt oben durch die Austrittsöffnung 66e, wie mit den Pfeilen 64b dargestellt, mit relativ geringer Flussgeschwindigkeit wieder aus dem Innenraum 66a aus. Im Innenraum 66a reduziert sich die Fliessgeschwindigkeit auf Grund des sich gegen oben erweiternden Querschnittes. Der Innenraum 66a bildet den ersten Strömungsraum. Ist der Durchmesser der Austrittsfläche 66e beispielsweise doppelt so gross wie der Durchmesser der Eintrittsfläche 66d, so entspricht die Geschwindigkeit an der Austrittsfläche 66e einem Viertel der Geschwindigkeit an der Eintrittsfläche 66d. Die durch die Fliessgeschwindigkeit bewirkte Auftriebskraft beträgt an der Austrittsfläche 66e noch einen Sechzehntel derjenigen an der Eintrittsfläche 66d. Das im Innenraum 66a angeordnete Gut 73 wird durch die nach oben strömende Flüssigkeit in einer Gleichgewichtslage gehalten, wobei sich die Schwebehöhe beziehungsweise die Gleichgewichtslage von Auftriebskraft und Schwerkraft auf Grund des Gewichtes und der Angriffsfläche des Guts von selbst einstellt.

Oberhalb des Flussleitmittels 66 ist eine Pumpe 65 angeordnet, welche einen ausserhalb des Behälters 62 angeordneten Eisenstator 65a und einen innerhalb des Behälters 62 angeordneten Rotor 65b umfasst. Ein Flügelrad 65c ist mit dem Rotor 65b fest verbunden. Eine derartige Vorrichtung umfassend einen Stator sowie einen mit magnetisch wirkenden Kräften gehaltenen und angetriebenen Rotor wird auch als lagerloser Motor bezeichnet, und ist einem Fachmann bekannt, beispielsweise aus der Druckschrift WO 96/31934.

Das Flügelrad 65c erzeugt die mit dem Pfeilen 64a, 64b, 64c dargestellte Kreisströmung. Zwischen dem Behälter 62 und dem Flussleitmittel 66 ist ein Innenraum 62e ausgebildet, auch als zweiter Strömungsraum bezeichnet, mit einer sich von oben nach unten erweiternden Querschnittsfläche. Die hat zur Folge, dass die in Strömungsrichtung 64c von oben nach unten strömende Flüssigkeit eine gegen unten abnehmende Fliessgeschwindigkeit aufweist.

Im Innenraums 62e ist unten ein ringförmiger Verteiler 67 angeordnet, durch welchen Luft oder Sauerstoff zur Begasung der Flüssigkeit 64 eingeleitet wird, welche innerhalb der Flüssigkeit 64 Luftblasen 68 bilden, welche aufzusteigen bestrebt sind. Durch die in Richtung 64c von oben nach unten strömende Flüssigkeit wird das Aufsteigen der Luftblasen 68 verzögert oder verhindert, was den Gasaustausch zur Flüssigkeit 64 fördert.

Der Behälter 62 ist aussen von einer ringförmigen Heizvorrichtung 69 umgeben. Der Innenraum des Behälters 62 wird über eine Zuleitung 67a und eine Ableitung 67b mit einer Nährflüssigkeit versorgt. Eine Messsonde 72 mit Sondenkopf 72a ermöglich beispielsweise eine Überwachung des pH-Wertes oder der Temperatur der Flüssigkeit 64.

Der in Figur 1 dargestellte Bioreaktor 61 weist den Vorteil auf, dass das Gut 73 über den einen grossen Durchmesser aufweisenden Verschluss 63 leicht zugänglich ist.

Fig. 1a zeigt eine perspektivische Darstellung des Flussleitmittels 66 mit Innenraum 66a.

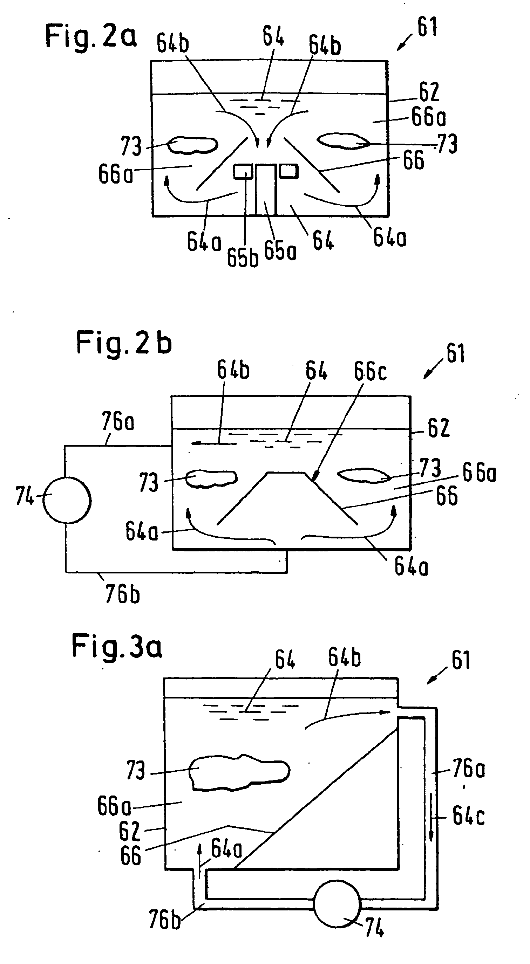

Fig. 2a zeigt schematisch einen Längsschnitt durch ein weiteres Ausführungsbeispiel eines Bioreaktors 61, welcher sich bezüglich dem in Fig. 1 dargestellten Beispiel dadurch unterscheidet, dass das Flussleitmittel 66 umgekehrt, das heisst mit einem sich gegen unten erweiternden Querschnitt angeordnet ist. Die Pumpe 65 umfassend den Eisenstator 65a und den rotierbaren Teil 65b mit Flügelrad 65c, bewirkt in der Flüssigkeit 64 eine Strömung in Richtung 64a, 64b. Der Innenraum 66a, in welchem die Flüssigkeit nach oben strömt, und in welchem das Gut 73 gehalten ist, befindet sich zwischen dem Flussleitmittel 66 und der Aussenwand des Behälters 62.

Fig. 2b zeigt schematisch einen Längsschnitt durch ein weiteres Ausführungsbeispiel eines Bioreaktors 61, welcher sich bezüglich dem in Fig. 2a dargestellten Beispiel dadurch unterscheidet, dass das Flussleitmittel 66 oben dicht ausgestaltet ist, und dass die Fluidpumpe 74 ausserhalb des Behälters 62 angeordnet ist, wobei die Pumpe 74 über Leitungen 76a, 76b fluidleitend mit dem Innenraum des Behälters 62 verbunden ist. Das in Richtung 64a strömende Fluid tritt von unten in den Innenraum 66a ein und umströmt das Gut 73.

Fig. 3a zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Das Flussleitmittel 66 ist nur auf der einen Innenseite des Behälters 62 sich gegen oben erweiternd ausgestaltet. Das Gut 73 wird durch die in Richtung 64a, 64b, 64c zirkulierende Flüssigkeit im Innenraum 66a in der Schwebe gehalten.

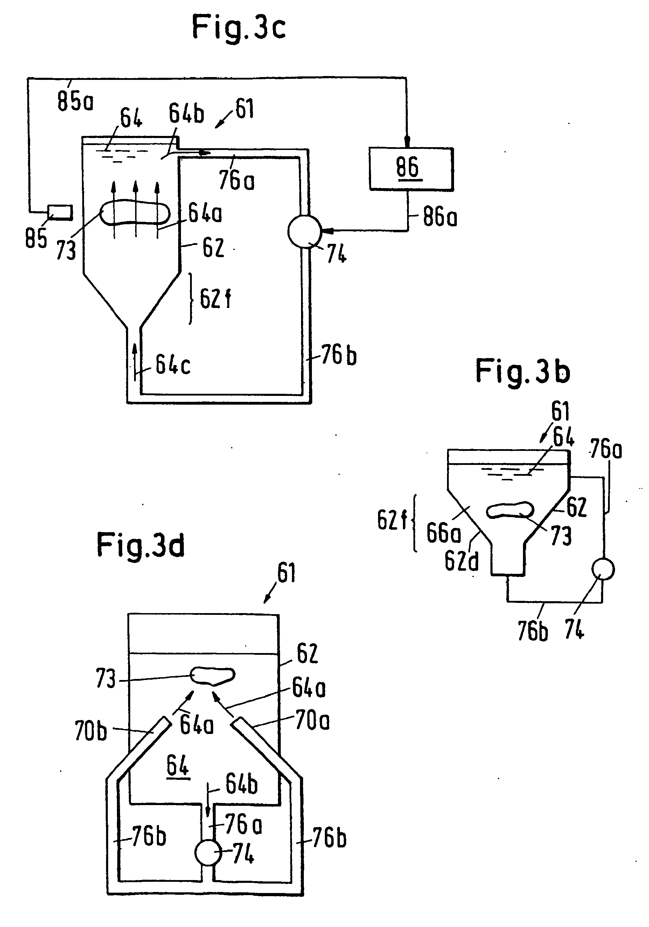

Fig. 3b zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Der Behälter 62 weist entlang eines Abschnitts 62f eine sich gegen oben erweiternde Behälterwand 62d auf. Entlang dieses Abschnittes 62f bildet sich eine Strömung mit einer sich gegen oben verringernden Fliessgeschwindigkeit auf, sodass der Innenraum 66a zum schwebenden Halten des Guts 73 entlang dieses Abschnittes 62f ausgebildet ist.

Fig. 3c zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Die Leitung 76a münden in einem sich gegen oben erweiternden Abschnitt 62f in den Behälter 62 ein. Nachfolgend ist ein zylinderförmig ausgestalteter Behälterabschnitt 62 angeordnet, innerhalb welchem sich eine lineare Strömung 64a ausbildet und innerhalb welchem das Gut 73 angeordnet ist. Mit einem Sensor 85 wird die Höhenlage des Gut 73 überwacht. Eine Regelvorrichtung 86 ist über eine elektrische Leitung 85a, 86a signalleitend mit dem Sensor 85 sowie der Pumpe 74 verbunden. Die Drehzahl der Pumpe 74 wird derart geregelt, dass das Gut 73 in Bereich des Sensors 85 verbleibt.

Fig. 3d zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Mehrere, beispielsweise drei Düsen 70a, 70b münden innerhalb des Behälters 62 auf das Gut 73 ausgerichtet, wobei die mit 64a dargestellte Strömungsrichtung eine gegen oben reduzierte Fliessgeschwindigkeit aufweist, sodass das Gut 73 von dieser Strömung getragen wird und selbstständig eine Gleichgewichtslage findet.

In allen in den Figuren 1 bis 3d dargestellten Bioreaktoren 61 wird das Gut 73 mit demselben Verfahren in einem schwebenden Zustand gehalten, nämlich dadurch, dass das Gut 73 mit einem Fluid beaufschlag wird, dessen Strömung entgegen der auf das Gut 73 wirkenden Schwerkraft wirkt, derart, dass das Gut 73 in der Schwebe gehalten wird. In den Ausführungsbeispielen gemäss den Figuren 1, 2a, 2b, 3a, 3b und 3d weist das Fluid im Innenraum 66a mit zunehmender Höhe eine geringere Strömungsgeschwindigkeit auf. Im Ausführungsbeispiel gemäss Fig. 3c wird die Geschwindigkeit des Fluides mit einem Sensor 85 in Abhängigkeit der Lage des Guts 73 geregelt.

Im Ausführungsbeispiel gemäss Fig. 1 wird innerhalb des Behälters 62 eine nach unten fliessende Strömung 64c erzeugt, wobei in diese Strömung 64c ein gasförmiges Fluid wie Luft oder Sauerstoff eingeleitet wird. Die Fliessgeschwindigkeit der Strömung 64c kann derart gewählt werden, dass das eingeleitete, gasförmige Fluid im Behälter 62 verlangsamt oder gar nicht mehr aufsteigt.

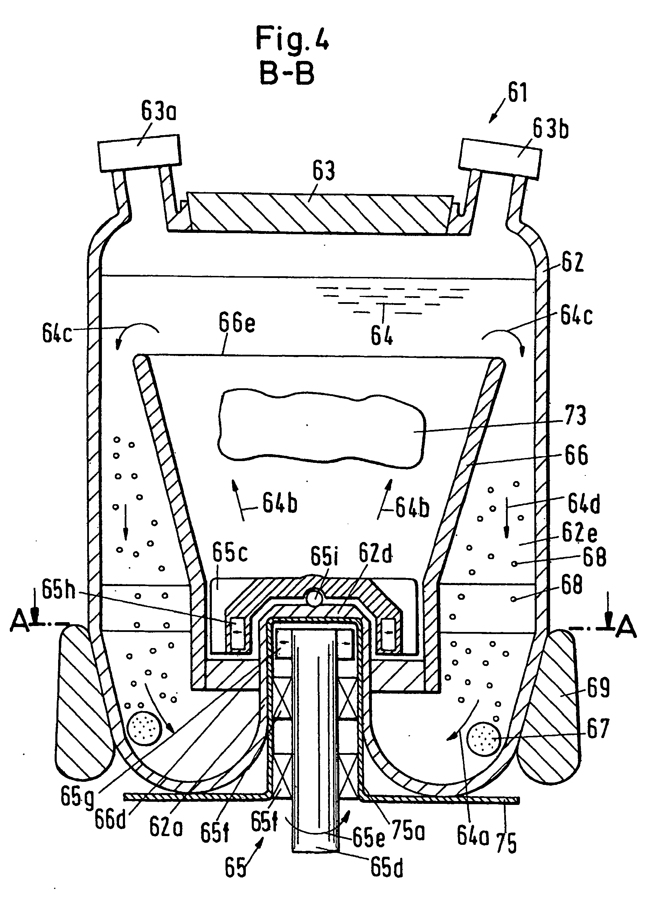

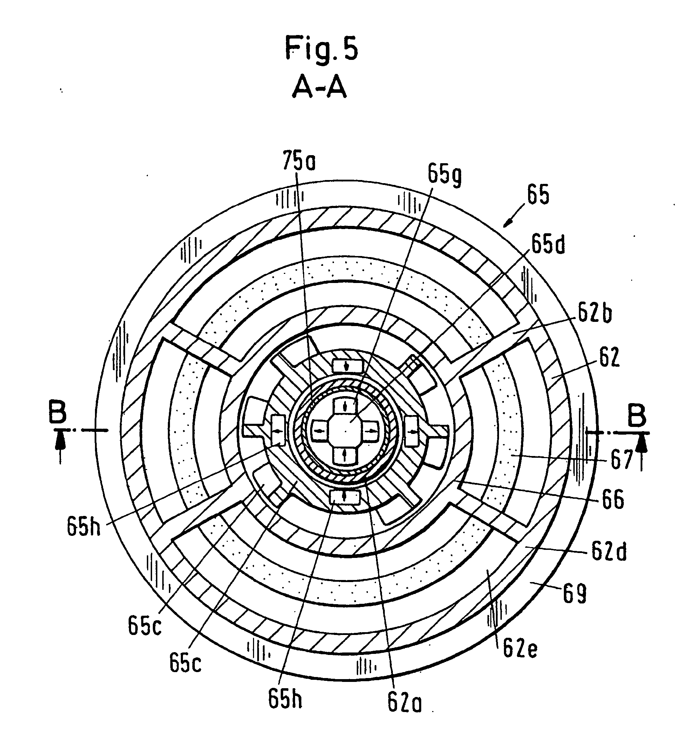

Fig. 4 zeigt mit einem Längsschnitt entlang der Linie B-B gemäss Fig. 5 ein weiteres Ausführungsbeispiel eines Bioreaktors 61. Ansonst ähnlich ausgestaltet wie der in Fig. 1 dargestellte Bioreaktor 61, ist im Bioreaktor 61 gemäss Fig. 4 die Pumpe 65 unten, im Bereich der Eintrittsöffnung 66d des Flussleitmittels 66 angeordnet. Innerhalb des Behälters 62 ist ein Flügelrad 65c drehbar auf einem Spurlager 65i angeordnet, wobei das Spurlager 65i auf der Behälterwand 62d aufliegt. Innerhalb des aus einem Kunststoff bestehenden Flügelrad 65c ist eine Mehrzahl über den Umfang verteilt angeordneter Permanentmagnete 65h eingegossen. Ausserhalb des Behälters 62 ist eine in Richtung 65e drehbar gelagerte Magnetkupplung angeordnet, welche zwei Lager 65f und einen ringförmigen Permanentmagneten 65g umfasst. Die rotierbare Welle 65d ist von einem nicht dargestellten Motor angetrieben. Eine Standvorrichtung 75 bildet einen Spalttopf 75a, welcher zylinderförmig ausgestaltet ist und zwischen den beiden Permanentmagneten 65g, 65h verlaufend angeordnet ist. Die Behälterwand 62d bildet beim Spalttopf 75a einen Spalttopfabschnitt 62a aus. Die magnetische Kupplung umfassend die Permanentmagnete 65h, 65i bewirkt, dass die Drehbewegung der rotierbaren Welle 65d auf das Flügelrad 65c übertragen wird, und dass das Flügelrad 65c bezüglich einem Kippen gehalten ist. Somit ist das Flügelrad 65c passiv magnetisch gehalten.

Der Behälter 62 sowie das darin drehbar gelagerte Flügelrad 65c ist vorzugsweise zur einmaligen Verwendung als Einmälprodukt ausgestaltet. Der Behälter 62 kann auf die Heizvorrichtung 69 sowie auf den Spalttopf 75a aufgesetzt werden, sodass der Behälter 62 sicher gehalten ist und das Flügelrad 65c über die drehbar gelagerte Magnetkupplung antreibbar ist.

Der Behälter 62 kann, wie in Fig. 4 dargestellt, zusätzliche Öffnungen 63a, 63b aufweisen, beispielsweise für Messsonden.

Fig. 5 zeigt einen Querschnitt entlang der Linie A-A gemäss Fig. 4. Im Zentrum ist die rotierbare Welle 65d angeordnet, an welcher vier beabstandet angeordnete Permanentmagnete 65g befestigt sind. Der Behälter 62 bildet mit dessen Behälterwand 62d einen Spalttopfabschnitt 62a aus. Zwischen dem Spalttopfabschnitt 62a und der rotierbaren Welle 65d mit Permanentmagnet 65g ist der Spalttopf 75a angeordnet. Der Spalttopfabschnitt 62a ist vom Flügelrad 65c umgeben, innerhalb welchem vier Permanentmagnete 65h angeordnet sind, wobei deren Polarisierung, mit Pfeilen dargestellt, derjenigen der Permanentmagnete 65g angepasst ausgerichtet ist. Das Flussleitmittel 66 ist über Fluidleitteile 62d mit der Aussenwand des Behälters 62 verbunden. Zwischen dem Flussleitmittel 66 und der Aussenwand des Behälters 62 ist der sich von oben nach unten erweiternde Strömungsraum 62e angeordnet. Zudem ist der ringförmig ausgestaltete Verteiler 67 dargestellt.

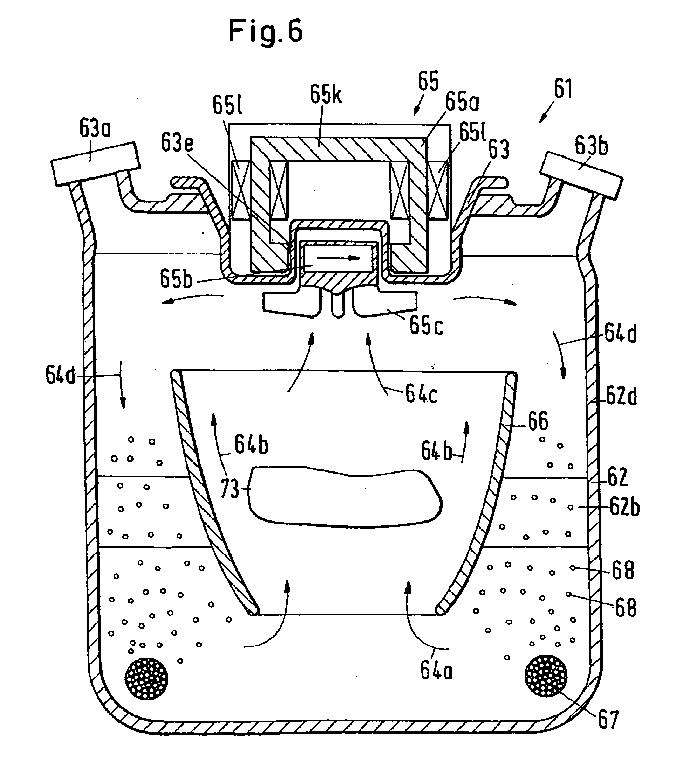

Fig. 6 zeigt einen Längsschnitt durch ein weiteres Ausführungsbeispiel eines Bioreaktors 61. Im Unterschied zu dem in Fig. 1 dargestellten Bioreaktor 61, ist im Bioreaktor 61 gemäss Fig. 6 die Pumpe 65 im Verschluss 63 angeordnet und als Zentrifugalpumpe ausgestaltet. Die Pumpe 65 ist als Spaltrohrmotor ausgestaltet und umfasst den fest angeordneten Eisenstator 65a sowie den berührungslos drehbar gelagerten, rotierbaren Teil 65b, ausgebildet als Permanentmagnet, welcher fest mit dem Flügelrad 65c verbunden ist. Der Eisenstator 65a umfasst ein Weicheisen 65k, welches von einer Mehrzahl von Spulen 651 umgeben ist. Die Spulen 65l sind derart angeordnet und ansteuerbar, dass der rotierbare Teil 65b berührungslos antreibbar und gehalten ist. Der Verschluss 63 weist einen Spalttopfabschnitt 63e auf, welcher im Spalt zwischen dem Eisenstator 65a und dem Permanentmagnet 65b angeordnet ist.

Eine derartige Vorrichtung umfassend einen Stator sowie einen mit magnetisch wirkenden Kräften gehaltenen und angetriebenen Rotor wird auch als Tempelmotor bezeichnet, und ist einem Fachmann bekannt, beispielsweise aus der Druckschrift WO 96/31934, insbesondere aus deren Figur 12.

Das Flussleitmittel 66 ist über Fluidleitteile 62b fest mit der Behälterwand 62d verbunden. Das Flussleitmittel 66 weist einen sich gegen oben bauchförmig erweiternden Querschnitt auf. Das Flussleitmittel 66 ist in einer Vielzahl weiterer Ausführungsformen ausgestaltbar, derart, dass sich eine gegen oben vergrössernde Querschnittsfläche ergibt.

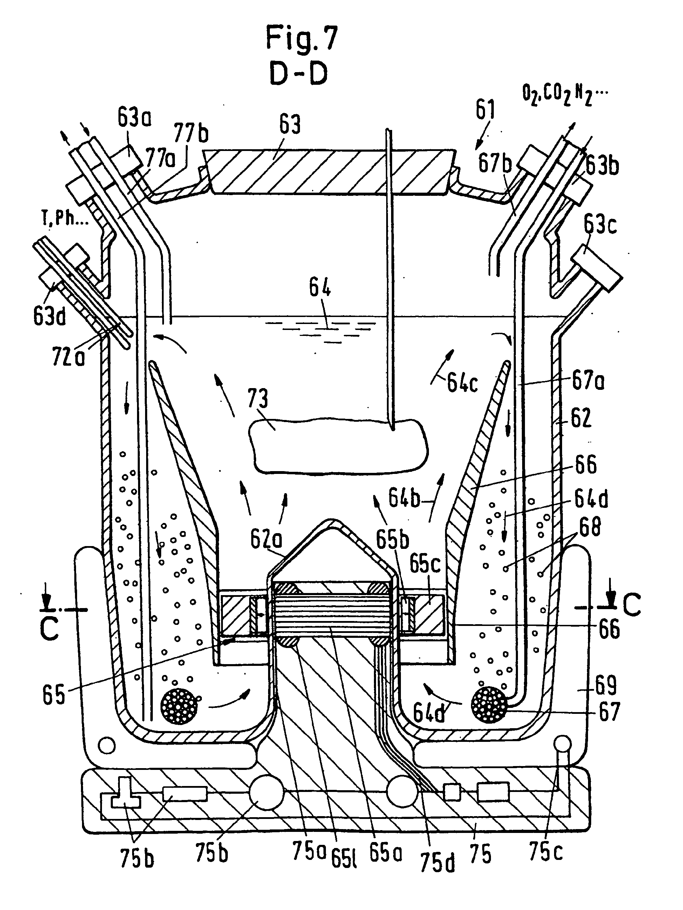

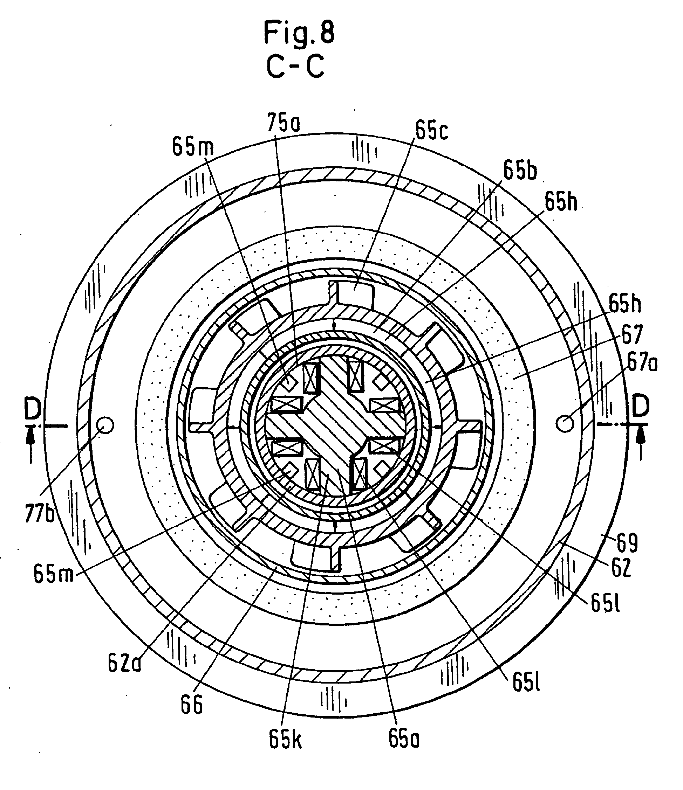

Fig. 7 zeigt mit einem Längsschnitt entlang der Linie D-D gemäss Fig. 8 ein weiteres Ausführungsbeispiel eines Bioreaktors 61. Im Unterschied zu dem in Fig. 4 dargestellten Bioreaktor 61 weist die Pumpe 65 ein vollständig magnetisch gelagertes und angetriebenes rotierbares Teil 65b mit Flügelrad 65c auf. Aus dem in Fig. 8 dargestellten Querschnitt entlang der Schnittlinie C-C ist der lagerlose Antrieb der Pumpe 65 im Detail dargestellt. Die Funktionsweise eines derartigen Antriebs ist beispielsweise in der Druckschrift WO 98/59406 offenbart. Der Eisenstator 65a ist als kreuzförmiges Blechpaket 65k ausgestaltet, an dessen Arme Spulen 65l angeordnet sind. Durch eine entsprechende Ansteuerung der Spulen 65I ist damit ein sich drehendes, magnetisches Feld erzeugbar. Das rotierbare Teil 65b umfasst vier in Umfangsrichtung angeordnete Permanentmagnete 65h, wobei zwei benachbarte Permanentmagnete 65h jeweils in entgegengesetzter Richtung polarisiert sind. Diese Permanentmagnete 65h sind im Flügelrad 65c bzw. in den Pumpenschaufeln 65c eingegossen bzw. gekapselt. Im Stator sind Sensoren 65m angeordnet, welche die Lage der Permanentmagnete 65h messen. In der Standvorrichtung 75 sind elektronische Komponenten 75b angeordnet, mit einer elektrischen Zuleitung 75d für die Spulen 65I des Motors sowie mit einer elektrischen Zuleitung 75c für die Heizung 69. Zudem sind elektrische Leitungen angeordnet, welche die Sensoren 65m mit den elektronischen Komponenten 75b verbindet. Die Spulen 65I werden derart angesteuert, dass das rotierbare Teil 65 mit Pumpenschaufeln 65c berührungslos gehalten und angetrieben ist. Die Pumpe 65 bildet eine Axialpumpe. Zwischen dem Eisenstator 65a und dem rotierbaren Teil 65b ist der Spalttopf 75a und der Spalttopfabschnitt 62a der Behälterwand 62d angeordnet.

Die Standvorrichtung 75 sowie die Heizung 69 bilden eine feste Unterlage und Halterung, in welche der Behälter 62 einführbar ist. Diese Anordnung weist den Vorteil auf, dass der Behälter 62 sehr einfach auf die Standvorrichtung 75 mit Heizung 69 gestellt werden kann, und die Axialpumpe 65 danach sofort betreibbar ist, ohne zusätzliche Handgriffe zu tätigen. Der Behälter 62 mit rotierbarem Teil 65b und Pumpenschaufeln 65c ist als Einmalprodukt ausgestaltet, wogegen die teuren Komponenten der Standvorrichtung 75 und der Heizung 69 belieb oft verwendbar sind. Zudem muss die Standvorrichtung 75 sowie die Heizung 69 nicht steril sein, sodass kein aufwendiger Reinigungsprozess erforderlich ist. Vorteile dieser Anordnung sind die Tatsachen, dass der Innenraum des Behälters 62 problemlos steril gehalten werden kann, dass der Behälter 62 kostengünstig herstellbar ist, und dass die Standvorrichtung 75 ohne aufwendigen Reinigungsprozess und somit kostengünstig betreibbar ist.

Im Behälter 62 gemäss Fig. 7 verlaufen durch den Verschluss 63b die Zu-und Ableitung 67a, 67b für Gase wie O₂, CO₂, N₂, wobei die Zuleitung 67a Fluid leitend mit dem ringförmigen Verteiler 67 verbunden ist. Durch den Verschluss 63a verlaufen die Zu- und Ableitung 77a, 77b für das Nährmedium. Zudem verlaufen durch den Verschluss 63d Sonden mit Sondenköpfen 72a, beispielsweise zur Messung von Temperatur oder pH-Wert.

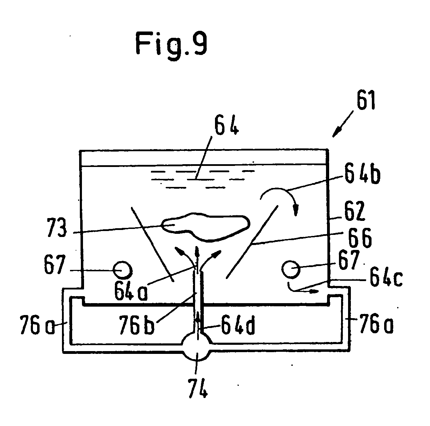

Fig. 9 zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Die Leitung 76b münden in den sich gegen oben erweiternden Abschnitt des Flussleitmittels 66 ein. Über die im Bodenbereich des Behälters 62 angeordneten Leitungen 76a wird das Fluid zur Fluidpumpe 74 geleitet, sodass das Fluid das mit Pfeilen 64a, 64b, 64c angedeutete Strömungsverhalten aufweist.

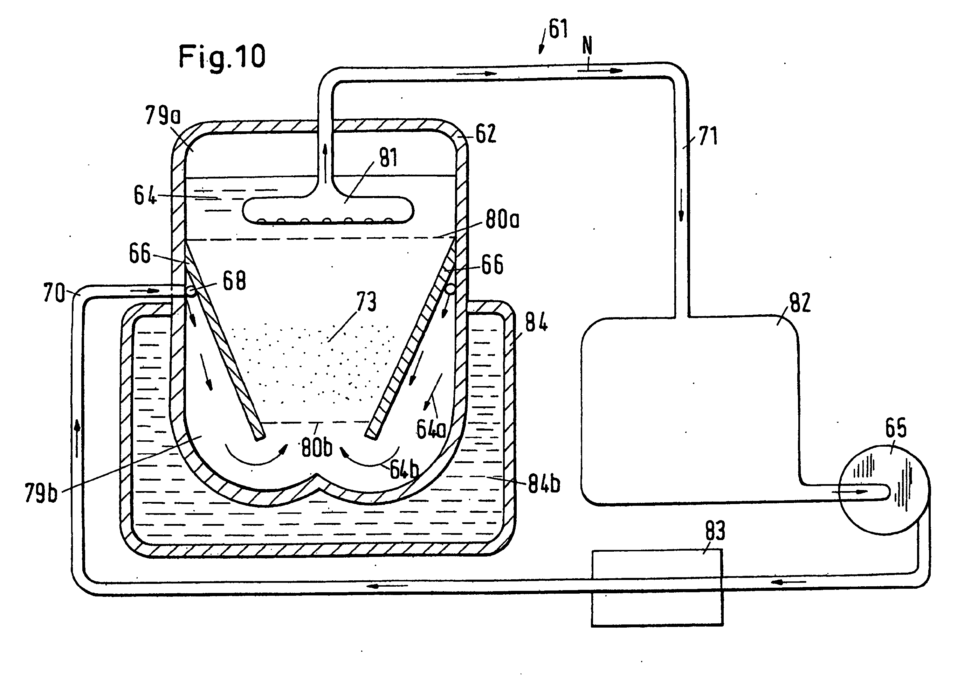

In Fig. 10 ist ein weiteres Ausführungsbeispiel des erfindungsgemässen Bioreaktors 61 dargestellt, in welchem Zellen gezüchtet werden können. Man erkennt den Reaktionsbehälter 62, der hier von einem weiteren Gefäss 84 umgeben ist, in welchem beispielsweise Wasser enthalten sein kann, um beispielsweise den Reaktionsbehälter 62 auf einer gewünschten Temperatur halten zu können. In dem Reaktionsbehälter ist ein kreiskegelstumpfförmiger Hohlkörper 66 angeordnet, der das Flussleitmittel 66 ausbildet, und der den Behälter 62 in eine obere Kammer 79a und eine untere Kammer 79b unterteilt. Der Mantel des kreiskegelstumpfförmigen Hohlkörpers 66 ist an seinem oberen Ende mit der Wand des Reaktionsbehälters 62 verbunden und verjüngt sich zum unteren Ende des Reaktionsbehälters hin. Die obere und untere Stirnfläche des Hohlkörpers 66 sind gas- und flüssigkeitsdurchlässig ausgebildet, und zwar derart, dass im Bereich der oberen und unteren Stirnfläche jeweils eine Membran 80a bzw. 80b angeordnet ist, welche gas- und flüssigkeitsdurchlässig ausgebildet ist. In dem Hohlraum, der zwischen den Membranen 80a und 80b eingeschlossen wird, können Zellträger, beispielsweise bestehend aus Kunststoff oder Keramik, mit Zellen 73 angeordnet sein, für welche die Membranen 80a und 80b undurchlässig sind. Die Zuführleitung 70 für die Nährlösung N mündet in der unteren Kammer 79b in einen ringförmigen Verteiler 68, welcher den Hohlkörper 66 umgibt. In der oberen Kammer 79a ist eine Absaugeinrichtung 81 vorgesehen, welche mit einer Abführleitung 71 verbunden ist, welche zu dem Reservoir 82 führt, wo die abgeführte Nährlösung N erneuert bzw. mit Nährstoffen angereichert werden kann. Zur Förderung der Nährlösung N ist eine Einmalpumpe 65 bzw. eine Pumpe mit Einmalteilen vorgesehen, welche beispielsweise als Zahnradpumpe oder als Zentrifugalpumpe ausgebildet sein kann.

Die von der Pumpe 65 aus dem Reservoir 82 geförderte Nährlösung N gelangt in einen Oxygenator 83, wo der Nährlösung N ein Gas wie beispielsweise Sauerstoff beigemischt oder Kohlendioxid entzogen werden kann. Die so mit Sauerstoff versetzte bzw. von Kohlendioxid befreite Nährlösung N gelangt dann im weiteren Verlauf in den ringförmigen Verteiler 68, der in der unteren Kammer 79b angeordnet ist. Mit Hilfe der Einmalpumpe 65 und der Absaugeinrichtung 81 wird eine Flüssigkeitsströmung erzeugt, welche durch die Pfeile 64a, 64b in Fig. 5 angedeutet ist. Im Bereich der Membran 80b ist die Strömungsgeschwindigkeit vergleichsweise hoch - sie nimmt dann aufgrund des kegelstumpfartig sich erweiternden Hohlkörpers 66 gegen oben ab. Durch eine geeignete Wahl der Strömungsparameter bzw. der Geometrie des Hohlkörpers 66 kann erreicht werden, dass die Zellen 73 bzw. das Gut 73 im Bereich zwischen den Membranen 80b und 80a in der Schwebe gehalten werden. Dies kann die Formation eines dreidimensionalen Zellverbunds bzw. Gewebeteils begünstigen. Bei diesem Ausführungsbeispiel erfolgt die Zuführung von Nährlösung N einerseits und von Gasen wie z.B. Sauerstoff andererseits nicht getrennt, sondern die Nährlösung N wird mit Sauerstoff versetzt, bevor sie mit Hilfe der Zuführleitung 70 und dem Verteiler 68 in den Behälter 62 eingebracht wird.

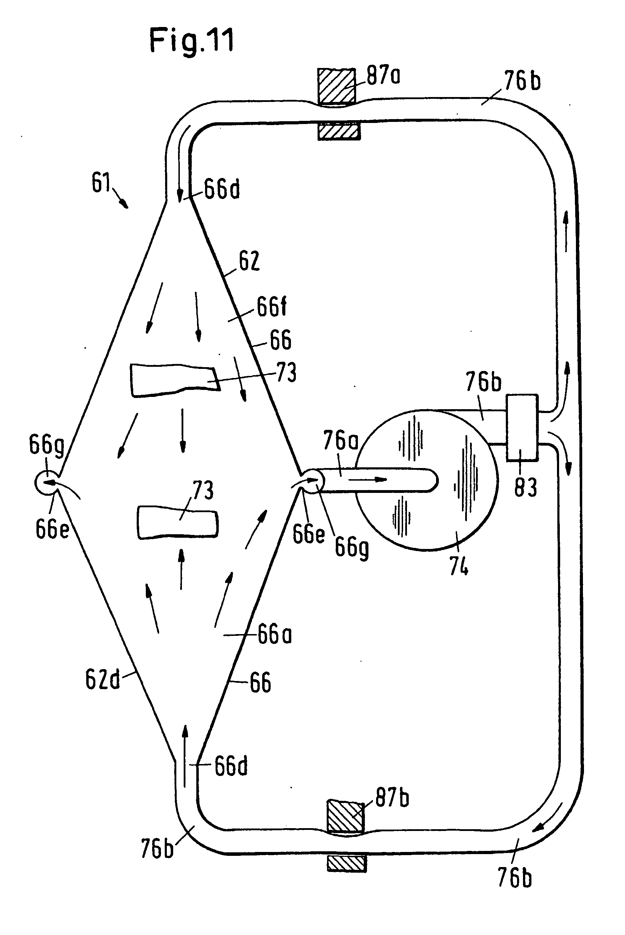

Fig. 11 zeigt in einem Längsschnitt schematisch ein weiteres Ausführungsbeispiel eines Bioreaktors 61. Dieser weist einen Behälter 62 auf mit einem ersten Strömungsraum 66a und einem darüber angeordneten zweiten Strömungsraum 66f. Diese beiden Strömungsräume 66a, 66f bilden einen gemeinsamen Innenraum, welcher oben und unten je eine Eintrittsöffnung 66d für das Fluid aufweist. Zwischen der oberen und unteren Eintrittsöffnung 66d ist eine ringförmige Austrittsöffnung 66e angeordnet, über welche das Fluid mittels eines ringförmigen Ableitkanals 66g und einer Fluidleitung 76a der Pumpe 74 zugeführt wird. Nach der Pumpe 74 führt die Fluidleitung 76b durch einen Oxigenator 83, worauf sich die Fluidleitung 76b in zwei Äste teilt, die das Fluid der oberen und/oder unteren Eintrittsöffnung 66d zuführen. Die in diesen Ästen strömende Fluidmenge ist über die erste und zweite Klemmvorrichtung 87a, 87b einstellbar bzw. ansteuerbar. Die Klemmvorrichtungen 87a, 87b erlauben den Durchmesser der Fluidleitung 76b zu verändern. Die Klemmvorrichtungen 87a, 87b können beispielsweise von Hand betätigbar sein, oder elektrische Antriebsvorrichtungen aufweisen, welche über nicht dargestellte Steuerleitungen mit einer übergeordneten Regelvorrichtung verbunden sind. In einer vorteilhaften Einstellung werden beide Äste der Fluidleitung 76b mit etwa derselben Fluidmenge durchströmt, sodass im ersten und zweiten Strömungsraum 66a, 66f etwa gleiche Strömungsverhältnisse, jedoch mit entgegengesetzter Richtung, auftreten, sodass das Gut 73 sowohl bei einer angreifenden Auftriebskraft als auch bei einer angreifenden Schwerkraft sicher innerhalb des Behälters 62 schwebend gehalten ist. Das Gut 73 kann somit ohne eine automatische Regelung in der Schwebe gehalten werden. Die Lage des Gutes 73 kann jedoch auch mit Hilfe einer automatischen Regelung überwacht und beeinflusst werden, indem die Lage des Gutes 73 mit einem nicht dargestellten Sensor erfasst wird. Sollte das spezifische Gewicht des Gutes 73 leichter als dasjenige des Fluides beziehungsweise der Nährlösung sein, so erfährt das Gut 73 einen Auftrieb. In diesem Falle wird das Fluid vermehrt über die obere Eintrittsöffnung 66d in den Behälter 62 eingeströmt, um im zweiten Strömungsraum 66f eine gegen unten gerichtete Fluidströmung zu bewirken, sodass das Gut 73 durch die entgegen der Auftriebskraft wirkende Fluidströmung in der Schwebe gehalten wird. Sollte sich das spezifische Gewicht des Gutes 73 mit der Zeit verändern und schwerer als dasjenige der Nährlösung werden, sodass auf das Gut 73 eine nach unten wirkende Schwerkraft wirkt, so wird das Fluid vermehrt der unteren Eintrittsöffnung 66d zugeführt, um im ersten Strömungsraum 66a eine nach oben gerichtete Fluidströmung zu erzeugen und dadurch eine Auftriebskraft auf das Gut 73 zu bewirken. Die geförderte Fluidmenge pro Zeiteinheit, und die Zuteilung der Teilmengen an die obere und/oder untere Eintrittsöffnung 66d erfolgt von Hand oder mit einer nicht dargestellten Regelungsvorrichtung derart, dass das Gut 73 ständig innerhalb des Behälters 62 in der Schwebe gehalten wird. Der Bioreaktor 61 kann in einer Vielzahl möglicher Formgebungen ausgestaltet sein, um ein darin befindliches Gut 73 sowohl bezüglich einer angreifenden Auftriebskraft als auch bezüglich einer angreifenden Schwerkraft durch entsprechend gewählte Fluidströme in der Schwebe zu halten.

## Patentansprüche

1. Bioreaktor (61) mit einem Behälter (62) für ein mit einem Fluid zu beaufschlagendes Gut (73), umfassend einen ersten Strömungsraum (66a) welchem ein strömendes Fluid zuführbar ist, wobei der erste Strömungsraum (66a) derart ausgestaltet ist, dass das darin von unten nach oben strömende Fluid mit zunehmender Höhe eine kleinere Geschwindigkeit aufweist, **dadurch gekennzeichnet, dass** die Fluidfördervorrichtung (65) ein Fluidfördermittel (65c), insbesondere ein Flügelrad, umfasst, dass das Fluidfördermittel (65c) innerhalb des Behälters (62) angeordnet ist, und dass die Fluidfördervorrichtung (65) einen Elektromotor umfasst, mit einem statischen Motorteil (65a) sowie einem rotierbaren Motorteil (65b), wobei das rotierbare Motorteil (65b) innerhalb des Behälters (62) angeordnet und magnetisch angetrieben ist, und das Fluidfördermittel (65c) mit dem rotierbaren Motorteil (65b) verbunden ist.

2. Bioreaktor (61) mit einem Behälter (62) für ein mit einem Fluid zu beaufschlagendes Gut (73), umfassend einen ersten Strömungsraum (66a) welchem ein strömendes Fluid zuführbar ist, sowie umfassend einen Sensor (85), welcher derart angeordnet und ausgestaltet ist, dass die Lage des beaufschlagten Gutes (73) im ersten Strömungsraum (66a) messbar ist, sowie umfassend eine mit dem ersten Strömungsraum (66a) fluidleitend verbundene Fluidfördervorrichtung (74), und umfassend eine Regelvorrichtung (86) welche mit dem Sensor (85) sowie der Fluidfördervorrichtung (74) Signal leitend verbunden ist, **dadurch gekennzeichnet, dass** die Fluidfördervorrichtung (65) ein Fluidfördermittel (65c), insbesondere ein Flügelrad, umfasst, dass das Fluidfördermittel (65c) innerhalb des Behälters (62) angeordnet ist, und dass die Fluidfördervorrichtung (65) einen Elektromotor umfasst, mit einem statischen Motorteil (65a) sowie einem rotierbaren Motorteil (65b), wobei das rotierbare Motorteil (65b) innerhalb des Behälters (62) angeordnet und magnetisch angetrieben ist, und das Fluidfördermittel (65c) mit dem rotierbaren Motorteil (65b) verbunden ist

3. Bioreaktor (61) nach Anspruch 1 oder 2, **dadurch gekennzeichnet, dass** der erste Strömungsraum (66a) sich von unten nach oben erweiternd ausgestaltet ist.

4. Bioreaktor (61) nach Anspruch 3, **dadurch gekennzeichnet, dass** der Behälter (62) einen Abschnitt (62f) mit einer sich gegen oben erweiternden Behälterwand (62d) aufweist, und dass dieser Abschnitt (62f) den ersten Strömungsraum (66a) ausbildet.

5. Bioreaktor (61) nach einem der Ansprüche 1 bis 4, **dadurch gekennzeichnet, dass** zumindest eine Fluidleitung (76b) in den ersten Strömungsraum (66a) mündet, vorzugsweise von unten oder bezüglich dem Strömungsraum (66a) seitlich angeordnet.

6. Bioreaktor (61) nach einem der Ansprüche 1 bis 5, **dadurch gekennzeichnet, dass** im Behälter (62) ein Flussleitmittel (66) angeordnet ist, welches den ersten Strömungsraum (66a) ausbildet, wobei das Flussleitmittel (66) derart ausgestaltet ist, dass sich der erste Strömungsraum (66a) von unten nach oben erweitert.

7. Bioreaktor (61) nach Anspruch 6, **dadurch gekennzeichnet, dass** das Flussleitmittel (66) als Hohlkörper (66b) ausgestaltet ist.

8. Bioreaktor (61) nach Anspruch 7, **dadurch gekennzeichnet, dass** der Hohlkörper (66b) einen sich gegen oben erweiternden Innenraum aufweist, welcher den ersten Strömungsraum (66a) ausbildet.

9. Bioreaktor (61) nach Anspruch 7, **dadurch gekennzeichnet, dass** der Hohlkörper (66b) eine sich gegen oben reduzierende Aussenkontur (66c) aufweist, und dass der Hohlkörper (66b) derart im Behälter (62) angeordnet ist, dass der sich von unten nach oben erweiternde erste Strömungsraum (66a) zwischen der Aussenkontur (66c) und der Behälterwand (62d) ausgebildet ist.

10. Bioreaktor (61) nach einem der Ansprüche 7 bis 9, **dadurch gekennzeichnet, dass** der Hohlkörper (66b) kreiskegelstumpfförmig ausgebildet ist.

11. Bioreaktor (61) nach einem der Ansprüche 1 bis 10, **dadurch gekennzeichnet, dass** der Behälter (62) oben zumindest eine verschliessbare Öffnung (62c) aufweist.

12. Bioreaktor (61) nach Anspruch 11, **dadurch gekennzeichnet, dass** die verschliessbare Öffnung (62c) eine Fläche von zumindest einem Viertel der Querschnittsfläche des Behälters (62) aufweist.

13. Bioreaktor (61) nach einem der Ansprüche 11 oder 12, **dadurch gekennzeichnet, dass** die verschliessbare Öffnung (62c) oberhalb des ersten Strömungsraums (66a) angeordnet ist.

14. Bioreaktor (61) nach einem der Ansprüche 1 bis 13, **dadurch gekennzeichnet, dass** der Elektromotor als Spaltrohrmotor ausgestaltet ist.

15. Bioreaktor (61) nach einem der Ansprüche 1 bis 14, **dadurch gekennzeichnet, dass** die Fluidfördervorrichtung (65) einen Magnetkupplungsantrieb umfasst, welcher zum Ankoppeln an das rotierbare Motorteil (65b) angepasst ausgestaltet ist.

16. Bioreaktor (61) nach einem der Ansprüche 1 bis 14, **dadurch gekennzeichnet, dass** der rotierbare Motorteil (65b) des Elektromotors zumindest bezüglich einem Freiheitsgrad mit aktiv oder passiv magnetisch wirkenden Mitteln gelagert ist.

17. Bioreaktor (61) nach Anspruch 16 ,**dadurch gekennzeichnet, dass** der statische und rotierbare Motorteil (65a, 65b) derart gegenseitig angepasst ausgestaltet sind, dass das rotierbare Motorteil (65b) vollständig magnetisch gelagert ist.

18. Bioreaktor (61) nach einem der vorhergehenden Ansprüche, **dadurch gekennzeichnet, dass** oberhalb des ersten Strömungsraumes (66a) ein zweiter Strömungsraum (66f) angeordnet ist, welcher derart ausgestaltet ist, dass das darin von oben nach unten strömende Fluid mit abnehmender Höhe eine kleinere Geschwindigkeit aufweist.

19. Bioreaktor (61) nach Anspruch 18, **dadurch gekennzeichnet, dass** der erste und der zweite Strömungsraum (66a,66f) einen gemeinsamen Innenraum bilden, welcher oben und unten eine Eintrittsöffnung (66d) für das Fluid aufweist, und welcher zwischen der oberen und unteren Eintrittsöffnung (66d) eine Austrittsöffnung (66e) aufweist.

20. Bioreaktor (61) nach Anspruch 19, **dadurch gekennzeichnet, dass** die Austrittsöffnung (66e) Fluid leitend mit einer Pumpe (74) verbunden ist, dass die Pumpe (74) Fluid leitend mit der oberen und unteren Eintrittsöffnung (66d) verbindbar ist, und dass die in die obere und/oder untere Eintrittsöffnung (66d) einströmende Fluidmenge ansteuerbar ist.

## Claims

1. A bioreactor (61) including a container (62) for a substance (73) to be acted upon with a fluid, including a first flow chamber (66a) to which a flowing fluid can be supplied, wherein the first flow chamber (66a) is designed in such a manner that fluid flowing from the bottom to the top has a smaller velocity with increasing height, **characterized in that** the fluid conveying apparatus (65) includes a fluid conveying means (65c), in particular a vaned wheel; and **in that** the fluid conveying means (65c) is arranged within the container (62) and **in that** the fluid conveying device (65) includes an electric motor having a static motor part (65a) as well as a rotatable motor part (65b), with the rotatable motor part (65b) being arranged within the container (62) and being magnetically driven and the fluid conveying means (65c) being connected to the rotatable motor part (65b).

2. A bioreactor (61) including a container (62) for a substance (73) to be acted upon with a fluid, including a first flow chamber (66a) to which a flowing fluid can be supplied, as well as including a sensor (85) which is arranged and designed in such a manner that the position of the substance (73) acted upon in the first flow chamber (66a) is measurable, and also including a fluid conveying apparatus (74) which is connected in a fluid guiding manner to the first flow chamber (66a), and including a regulation apparatus (86) which is connected to the sensor (85) and to the fluid conveying apparatus (74) in a signal guiding manner, **characterized in that** the fluid conveying apparatus (65) includes a fluid conveying means (65c), in particular a vaned wheel; and **in that** the fluid conveying means (65c) is arranged inside the container (62) and **in that** the fluid conveying apparatus (65) includes an electric motor having a static motor part (65a) and a rotatable motor part (65b), with the rotatable motor part (65b) being arranged inside the container (62) and being magnetically driven and the fluid conveying means (65c) being connected to the rotatable motor part (65b).

3. A bioreactor (61) in accordance with claim 1 or claim 2, **characterized in that** the first flow chamber (66a) is designed to widen upwardly.

4. A bioreactor (61) in accordance with claim 3, **characterized in that** the container (62) has a section (62f) with a container wall (62d) which widens upwardly; and **in that** this section (62f) forms the first flow chamber (66a).

5. A bioreactor (61) in accordance with any one of the claims 1 to 4, **characterized in that** at least one fluid line (76b) opens into the first flow chamber (66a), preferably from below or arranged laterally with respect to the flow chamber (66a).

6. A bioreactor (61) in accordance with any one of the claims 1 to 5, **characterized in that** at least one fluid guiding means (66) is arranged in the container (62) which forms the first flow chamber (66a), with the fluid guiding means (66) being designed such that the first flow chamber (66a) widens upwardly.

7. A bioreactor (61) in accordance with claim 6, **characterized in that** the fluid guiding means (66) is designed as a hollow body (66b).

8. A bioreactor (61) in accordance with claim 7, **characterized in that** the hollow body (66b) has an inner space which widens upwardly and which forms the first flow chamber (66a).

9. A bioreactor (61) in accordance with claim 7, **characterized in that** the hollow body (66b) has an upwardly reducing outer contour (66c); and **in that** the hollow body (66b) is arranged in the container (62) in such a manner that the first flow chamber (66a), which widens upwardly, is formed between the outer contour (66c) and the container wall (62d).

10. A bioreactor (61) in accordance with any one of the claims 7 to 9, **characterized in that** the hollow body (66b) is formed in the shape of a truncated circular cone.

11. A bioreactor (61) in accordance with any one of the claims 1 to 10, **characterized in that** the container (62) has at least one closeable opening (62c) above.

12. A bioreactor (61) in accordance with claim 11, **characterized in that** the closeable opening (62c) has a surface of at least a quarter of the cross-sectional area of the container (62).

13. A bioreactor (61) in accordance with any one of the claims 11 or 12, **characterized in that** the closeable opening (62c) is arranged above the first flow chamber (66a).

14. A bioreactor (61) in accordance with any one of the claims 1 to 13, **characterized in that** the electric motor is designed as a split tube motor.

15. A bioreactor (61) in accordance with any one of the claims 1 to 14, **characterized in that** the fluid conveying apparatus (65) includes a magnetic coupling drive which is designed to be adapted for coupling to the rotatable motor part (65b).

16. A bioreactor (61) in accordance with any one of the claims 1 to 14, **characterized in that** the rotatable motor part (65b) of the electric motor is journalled at least with respect to one degree of freedom with actively or passively magnetically acting means.

17. A bioreactor (61) in accordance with claim 16, **characterized in that** the static and rotatable motor part (65a, 65b) are designed to be mutually matched in such a manner that the rotatable motor part (65b) is completely magnetically journalled.

18. A bioreactor (61) in accordance with anyone of the preceding claims, **characterized in that** a second flow chamber (66f) is arranged above the first flow chamber (66a) and is designed in such a manner that the fluid flowing from the top to the bottom therein has a smaller velocity with decreasing height.

19. A bioreactor (61) in accordance with claim 18, **characterized in that** the first flow chamber and the second flow chamber (66a, 66f) form a common inner space which has an inlet opening (66d) for the fluid at the top and at the bottom and which has an outlet opening (66e) between the upper and lower inlet opening (66d).

20. A bioreactor (61) in accordance with claim 19, **characterized in that** the outlet opening (66e) is connected in fluid conducting manner to a pump (74), **in that** the pump (74) is connectable in fluid conducting manner to the upper and lower inlet opening (66d) and **in that** the quantity of fluid flowing into the upper and/or lower inlet opening (66d) can be controlled.

## Revendications

1. Bioréacteur (61) avec un contenant (62) pour un produit (73) à charger par un fluide, comprenant une première enceinte d'écoulement (66a) à laquelle peut être amené un fluide coulant, où la première enceinte d'écoulement (66a) est configurée de telle sorte que le fluide s'écoulant dans celle-ci depuis le bas vers le haut présente, au fur et à mesure que la hauteur augmente, une plus petite vitesse, **caractérisé en ce que** le dispositif de convoyage de fluide (65) comprend un moyen de convoyage de fluide (65c), en particulier une roue à ailettes, **en ce que** le moyen de convoyage de fluide (65c) est disposé à l'intérieur du contenant (62), et **en ce que** le dispositif de convoyage de fluide (65) comprend un moteur électrique, avec une partie de moteur statique (65a) et une partie de moteur tournante (65b), où la partie de moteur tournante (65b) est disposée à l'intérieur du contenant (62) et est entraînée magnétiquement, et **en ce que** le moyen de convoyage de fluide (65c) est relié à la partie de moteur tournante (65b).

2. Bioréacteur (61) avec un contenant (62) pour un produit (73) à charger par un fluide, comprenant une première enceinte d'écoulement (66a) à laquelle peut être amené un fluide coulant, et comprenant un capteur (85) qui est disposé et configuré de telle sorte que la position du produit chargé (73) dans la première enceinte d'écoulement (66a) peut être mesurée, et comprenant un dispositif de convoyage de fluide (74) relié à la première enceinte d'écoulement (66a) en conduisant le fluide, et comprenant un dispositif de régulation (86) qui est relié au capteur (85) et au dispositif de convoyage de fluide (74) en conduisant les signaux, **caractérisé en ce que** le dispositif de convoyage de fluide (65) comprend un moyen de convoyage de fluide (65c), en particulier une roue à ailettes, **en ce que** le moyen de convoyage de fluide (65c) est disposé à l'intérieur du contenant (62), et **en ce que** le dispositif de convoyage de fluide (65) comprend un moteur électrique, avec une partie de moteur statique (65a) et une partie de moteur tournante (65b), où la partie de moteur tournante (65b) est disposée à l'intérieur du contenant (62) et est entraînée magnétiquement, et **en ce que** le moyen de convoyage de fluide (65c) est relié à la partie de moteur tournante (65b).

3. Bioréacteur (61) selon la revendication 1 ou 2, **caractérisé en ce que** la première enceinte d'écoulement (66a) est réalisée de façon à s'élargir du bas vers le haut.

4. Bioréacteur (61) selon la revendication 3, **caractérisé en ce que** le contenant (62) présente une section (62f) avec une paroi de contenant (62d) s'élargissant vers le haut, et **en ce que** cette section (62f) forme la première enceinte d'écoulement (66a).

5. Bioréacteur (61) selon l'une des revendications 1 à 4, **caractérisé en ce qu'**au moins une conduite de fluide (76b) débouche dans la première enceinte d'écoulement (66a), de préférence depuis le bas ou est disposée latéralement relativement à l'enceinte d'écoulement (66a).

6. Bioréacteur (61) selon l'une des revendications 1 à 5, **caractérisé en ce qu'**est disposé dans le contenant (62) un moyen de guidage de fluide (66) qui forme la première enceinte d'écoulement (66a), où le moyen de guidage de fluide (66) est réalisé de telle sorte que la première enceinte d'écoulement (66a) s'élargit du bas vers le haut.

7. Bioréacteur (61) selon la revendication 6, **caractérisé en ce que** le moyen de guidage d'écoulement (66) est réalisé comme corps creux (66b).

8. Bioréacteur (61) selon la revendication 7, **caractérisé en ce que** le corps creux (66b) présente un espace intérieur s'élargissant vers le haut qui forme la première enceinte d'écoulement (66a).

9. Bioréacteur (61) selon la revendication 7, **caractérisé en ce que** le corps creux (66b) présente un contour extérieur (66c) se réduisant vers le haut, et **en ce que** le corps creux (66b) est disposé de telle sorte dans le contenant (62) que la première enceinte d'écoulement (66a) s'élargissant depuis le bas vers le haut est réalisée entre le contour extérieur (66c) et la paroi de contenant (62d).

10. Bioréacteur (61) selon l'une des revendications 7 à 9, **caractérisé en ce que** le corps creux (66b) est réalisé en forme de cône tronqué circulaire.

11. Bioréacteur (61) selon l'une des revendications 1 à 10, **caractérisé en ce que** le contenant (62) présente en haut au moins une ouverture (62c) pouvant être fermée.

12. Bioréacteur (61) selon la revendication 11, **caractérisé en ce que** l'ouverture pouvant être fermée (62c) présente une face d'au moins un quart de la face en section transversale du contenant (62).

13. Bioréacteur (61) selon l'une des revendications 11 ou 12, **caractérisé en ce que** l'ouverture (62c) pouvant être fermée est disposée au-dessus de la première enceinte d'écoulement (66a).

14. Bioréacteur (61) selon l'une des revendications 1 à 13, **caractérisé en ce que** le moteur électrique est réalisé sous forme de moteur à gaine.

15. Bioréacteur (61) selon l'une des revendications 1 à 14, **caractérisé en ce que** le dispositif de convoyage de fluide (65) comprend un entraînement d'embrayage magnétique qui est conçu pour le couplage à la partie de moteur tournante (65b).

16. Bioréacteur (61) selon l'une des revendications 1 à 14, **caractérisé en ce que** la partie de moteur tournante (65b) du moteur électrique, au moins relativement à un degré de liberté, est logée par des moyens à effet magnétique actif ou passif.

17. Bioréacteur (61) selon la revendication 16, **caractérisé en ce que** les parties de moteur statique et tournante (65a, 65b) sont adaptées mutuellement de façon que la partie de moteur tournante (65b) est logée entièrement magnétiquement.

18. Bioréacteur (61) selon l'une des revendications précédentes, **caractérisé en ce qu'**il est disposé au-dessus de la première enceinte d'écoulement (66a) une deuxième enceinte d'écoulement (66f), qui est réalisée de telle sorte que le fluide s'écoulant dans celle-ci du haut vers le bas présente, au fur et à mesure que la hauteur diminue, une vitesse plus petite.

19. Bioréacteur (61) selon la revendication 18, **caractérisé en ce que** les première et deuxième enceintes d'écoulement (66a, 66f) forment un espace intérieur commun qui présente en haut et en bas une ouverture d'entrée (66d) pour le fluide, et qui présente entre les ouvertures d'entrée supérieure et inférieure (66d) une ouverture de sortie (66e).

20. Bioréacteur (61) selon la revendication 19, **caractérisé en ce que** l'ouverture de sortie (66e) est reliée à une pompe (74) de manière à conduire le fluide, **en ce que** la pompe (74) peut être reliée de manière à conduire le fluide aux ouvertures d'entrée supérieure et inférieure (66d), et **en ce que** la quantité de fluide affluant dans l'ouverture d'entrée supérieure et/ou inférieure (66d) peut être commandée.
